# EUROPEAN PATENT APPLICATION

(11) **EP 1 291 359 A1**
(43) Date of publication of application: **12.03.2003**
(21) Application number: 02255718.5
(22) Date of filing: 15.08.2002
(51) Int. Cl.: C07K 14/705, G01N 33/68

(54) **Amino acid sequence motifs characteristic of GPCR subfamilies, and methods for GPCR characterization**

(30) Priority: 31.08.2001 US 316660 P
(71) Applicant: Pfizer Products Inc., Groton, Connecticut 06340 (US)
(72) Inventor: Huang, Enoch So-Wei, Pfizer Discovery, Cambridge, Massachusetts 02139 (US)
(74) Representative: England, Peter Michael

(57) **Abstract**

The invention relates to the field of bioinformatics. More particularly, the invention relates to methods of identifying sequence motifs characteristic of G-protein-coupled receptor (GPCR) subfamilies and using these motifs, for example, to classify "orphan GPCRs." The invention provides methods of classifying "orphan GPCRs" that focus on amino acid residue similarities for ligand-binding positions. The methods use optimized sequence alignments and avoid mechanical calculation of scores and cutoffs. In particular, a sequence motif characteristic of a GPCR subfamily that binds a certain ligand type is determined, and "orphan GPCRs" having the sequence motif are assigned to the subfamily.

## Description

### FIELD OF THE INVENTION

The present invention relates to bioinformatics, and specifically, to the use of computational biology to identify sequence motifs. These sequence motifs, which are characteristic of Class A (rhodopsin-like) G-protein coupled receptor (GPCR) subfamilies, can be used, for example, to classify "orphan GPCRs" into such subfamilies or receptor subtypes rendering such "de-orphanated" receptors more readily available for drug discovery.

### BACKGROUND OF THE INVENTION

Fundamental to pharmaceutical research is the provision of targets against which pharmaceuticals can be developed. GPCRs are precedented drug targets. In fact, several of the major drugs currently on the market were designed against particular GPCRs. GPCRs have been grouped into various classes and subfamilies based on their sequence homology, structural features, biological functions, and ligand-binding types. There are also numerous "orphan GPCRs" whose classes, subfamilies, and functions remain unknown. The determination of the subfamilies, and thus the ligand-binding types, of these "orphan GPCRs" will better position them for use as novel drug discovery targets. Clearly, it would be a rather resource intensive exercise to "de-orphanate" each of these GPCRs by routine screening against, e.g., compound libraries comprising ligands known to bind to specific GPCR subfamilies, as well as against ligands not specifically known to bind to specific GPCR subfamilies but might.

The field of computational biology or bioinformatics has responded to this need for a less resource intensive and more efficient way to assign specific receptor subtypes to "orphan GPCRs," e.g., by providing protein pattern databases based on sequence alignments (e.g., PROSITE patterns: encode single short motifs; PRINTS fingerprints: encode groups of motifs in the form of fingerprints that differentiate between regions of sequence that characterize the family and receptor subtype; PROSITE profiles and Pfam: utilize almost the complete sequence).

Most computational strategies for identifying specific receptor subtypes have focused on searching sequence databases, e.g., using commonplace alignment or sequence similarity tools such as BLAST, where these databases generally comprise characteristic protein family signatures, sequences, or profiles. Central to the use of this tool, and the attendant deconvolution of the data provided as a result of using the tool, is an understanding that BLAST reveals generic similarities but does not reveal individual family traits such as specific ligand-binding motifs; hence, BLAST can be a blunt tool. Likewise, PRINTS can, as the case may be, be too sharp of a tool, in that, not every residue identified as part of a conserved motif using PRINTS may be necessary for ligand binding, function, and the like.

As mentioned above, additional approaches for identifying GPCR subtypes have been taken. For example, sequence alignments can be created manually for each of the different superfamilies, and for the subfamilies and receptor subtypes, and this information, e.g., regions of similarity and difference, can be used to construct a range (or hierarchy) of discriminatory "fingerprints" or family signature (i.e., groups of conserved motifs). Generally, these conserved regions are functionally and/or structurally important regions within a protein family, e.g., transmembrane domains, ligand-binding sites, and similar). The ability of this tool to discriminate to the subtype level enables the identification of the specific residues involved in ligand-binding, G-protein coupling, and similar.

Notwithstanding the above, many GPCRs still remain "orphans." This is due, in large part, to the fact that the exact nature of ligand binding to GPCRs has remained difficult to ascertain, e.g., only one high-resolution GPCR structure, that of rhodopsin, is currently available. See, Palczewski et *al.*, *Science* 289:739-745 (2000). Using the existing methodology, as the overall similarity between a query sequence and a reference sequence or model decreases, automated methods have difficulty providing an accurate alignment on which to base residue comparisons. Alignment accuracy can be validated only by known structural correspondences between the query and reference sequences. Because only the rhodopsin structure is known, structure-based alignment is next to impossible for GPCR sequence comparisons. Thus, the existing computational methods of GPCR classification may produce inaccurate results by performing comparisons based on incorrect sequence alignments.

For example, as discussed above, a tool such as BLAST may find a region of high local similarity between the query and reference sequences, and reward that segment with a high score, while in fact the respective amino acids aligned by BLAST do not correspond to equivalent positions in the sequences. The true degree of relatedness between the pair of proteins is therefore masked by the irrelevant segment of local similarity. Alignment inaccuracies may be erroneously compounded where used as a basis for clustering-based approaches. Thus, there exists a need in the art for improved methods of classifying "orphan GPCRs," where such methods are less likely to generate results based on incorrect sequence alignments.

The existing computational methods for classifying GPCRs also suffer from a dependence on scores and cutoffs. The user must decide what score is required to classify a GPCR in a given subfamily. Score cutoffs balance sensitivity against specificity: an overly stringent cutoff may miss true positives, while an overly lenient cutoff may create false positives. There exists a need in the art for improved methods of classifying "orphan GPCRs" that are not dependent on scores and cutoff values that are inherently subject to error.

Thus, there exists a need in the art for improved methods of classifying "orphan GPCRs" that, given a family level similarity, evaluate the residue properties at the ligand-binding positions to predict subfamily membership. Ideally, such methods would identify sequence motifs required for ligand binding in any given GPCR subfamily, such that "orphan GPCRs" possessing the necessary motifs could be assigned to subfamilies and receptor subtypes and, as such, would not depend upon the above described scores and cutoffs.

The present invention provides improved methods that overcome the limitations in the art by providing GPCR subfamily sequence motifs. These motifs can be used to classify "orphan GPCRs," and to either further validate, or "correct" erroneous classification of, previously "de-orphanated" GPCRs. The "de-orphanated" GPCRs of the present invention, having been thus assigned to specific subfamilies using the methods of this invention, can be further explored where so desired, e.g., by performing suitable *in vitro* functional assays. The present motifs and methods employing the motifs enable a quicker assignation of "orphan GPCRs" to the correct subfamilies and receptor subfamilies, thus lessening the cost of, and improving the efficiency of, the provision of new targets for drug discovery which may expedite new medicines to the market.

### SUMMARY OF THE INVENTION

The present invention relates to sequence motif characteristics of GPCR subfamilies that bind particular ligand types. The present invention also relates to methods for identifying such characteristics. The invention further relates to methods employing such characteristics to assign "orphan GPCRs" to their rightful subfamilies or receptor subtypes.

In a first aspect, the present invention provides methods for determining amino acid sequence motifs characteristic of GPCR subfamilies.

Accordingly, such methods of the first aspect fundamentally include the steps, in sequence, of: (a) manually aligning amino acid sequences of members of the selected GPCR subfamily to create a subfamily alignment, (b) comparing the subfamily alignment with a known GPCR superfamily alignment, and (c) identifying at least one conserved position in the subfamily that is not conserved in the superfamily assignment, thus providing at least one distinguishing characteristic of the subfamily with respect to the superfamily.

In a preferred embodiment of the first aspect, a conserved position is located on an extracellular portion of the GPCRs of the subfamily. Preferred extracellular portions include the N-terminal domain, an extracellular loop, an extracellular portion of a helix, and a transmembrane helix.

In another preferred embodiment of the first aspect, a conserved position is occupied by a polar or an aromatic amino acid, where the polar amino acid is either charged or uncharged. Preferred aromatic amino acids include phenylalanine, tyrosine, tryptophan, and histidine.

In a second aspect, the present invention provides methods for determining amino acid sequence motifs characteristic of GPCR subfamilies, where the subfamily members interact with members of the ligand family through the identified motifs.

Accordingly, such methods of the second aspect fundamentally include the steps, in sequence, of: (a) manually aligning amino acid sequences of members of the selected GPCR subfamily to create a subfamily alignment, (b) comparing the subfamily alignment with a known GPCR superfamily alignment, (c) identifying at least one conserved position in the subfamily that is not conserved in the superfamily assignment, (d) identifying at least one common feature (e.g., a common chemical moiety) in members of the selected ligand family, and (e) determining if a binding interaction exists between the conserved position in the subfamily of (c) and the common feature of (d), where the presence of a binding interaction indicates that the conserved position of the subfamily is part of the sequence motif characteristic of the subfamily.

In a preferred embodiment of the second aspect, a conserved position is located on an extracellular portion of the GPCRs of the subfamily. Preferred extracellular portions include the N-terminal domain, an extracellular loop, an extracellular portion of a helix, and a transmembrane helix.

In another preferred embodiment of the second aspect, a conserved position is occupied by a polar or an aromatic amino acid, where the polar amino acid is either charged or uncharged. Preferred aromatic amino acids include phenylalanine, tyrosine, tryptophan, and histidine.

In another preferred embodiment of the second aspect, the members of the ligand family are identified by a common property. Preferred common properties include atomic composition and connectivity, electronic configuration (e.g., charge distribution, aromaticity, and similar), hydrophobicity, molecular weight, polarity, products of a common biochemical pathway or process, and shape (e.g., stereochemistry).

In yet another embodiment of the second aspect, a common chemical moiety of the ligand family is selected from the group consisting of the chemical moieties characteristic of amines (bioamines), peptides, lipids, melatonins, nucleotides, olfactory ligands, and opsins. Preferred common chemical moieties include an amino group, a carboxylate, and a phosphate group.

In a further embodiment of the second aspect, the ligand family is selected from ligand families that interact with GPCRs. Preferred ligand families include amines, peptides, lipids, melatonins, nucleotides, olfactory ligands, and opsins. Particularly preferred ligand families include amines, peptides, lipids, and nucleotides. Preferred peptides include opioids, neuropeptides, and proteins. Preferred proteins include chemokines. Preferred chemokines include complement proteins. Preferred lipids include eicosanoids, and sphingolipids. Preferred eicosanoids include leukotrienes and prostanoids.

In yet a further embodiment of the second aspect, the ligand family is amines. In a preferred embodiment of the second aspect wherein the ligand family is amines, the first conserved portion is a conserved aspartic acid residue located seventeen positions closer to the N-terminus of the GPCR than a conserved sequence consisting of aspartic acid, arginine, and tyrosine located at the C-terminus of the third transmembrane helix (TM3), and the second conserved position is an aromatic residue located ten positions closer to the N-terminus of the GPCR than a conserved proline in the seventh transmembrane helix (TM7). Preferred aromatic residues include tryptophan.

In a third aspect, the present invention provides methods of determining whether an "orphan GPCR" belongs to a GPCR subfamily.

Accordingly, such methods of the third aspect fundamentally include the steps, in sequence, of: (a) manually aligning amino acid sequences of members of the selected GPCR subfamily to create a subfamily alignment, (b) comparing the subfamily alignment with a known GPCR superfamily alignment, (c) identifying at least one conserved position in the subfamily that is not conserved in the superfamily assignment, and (d) determining whether the "orphan GPCR" comprises the subfamily's conserved position, thus identifying the "orphan GPCR" as a member of the subfamily.

In a fourth aspect, the present invention provides methods of determining whether an "orphan GPCR" belongs to a GPCR subfamily, where the subfamily members interact with members of the ligand family through the identified motifs.

Accordingly, such methods of the second aspect fundamentally include the steps, in sequence, of: (a) manually aligning amino acid sequences of members of the selected GPCR subfamily to create a subfamily alignment, (b) comparing the subfamily alignment with a known GPCR superfamily alignment, (c) identifying at least one conserved position in the subfamily that is not conserved in the superfamily assignment, (d) identifying at least one common feature (e.g., a common chemical moiety) in members of the selected ligand family, (e) determining if a binding interaction exists between the conserved position in the subfamily of (c) and the common feature of (d), where the presence of a binding interaction indicates that the conserved position of the subfamily is part of the sequence motif characteristic of the subfamily, and (f) determining whether the "orphan GPCR" comprises the sequence motif characteristic of the subfamily. As those skilled in the art will appreciate, the presence of a binding interaction can be shown by many conventional methods, e.g., crystallizing the receptor with the ligand bound, and/or site-directed mutagenesis (of the GPCR or the ligand), as described specifically herein. Such a binding interaction can be determined by, for example, interacting a GPCR with a member of a ligand family under conditions favoring ligand binding to the GPCR, exposing the GPCR/ligand complex to conditions favoring crystallization of the complex, and identifying a point of interaction between the GPCR and the member of the ligand family by examining the crystallized complex.

The present invention also provides methods of screening compound libraries against the "de-orphanated GPCRs," e.g., to identify modulators (such as, agonists and antagonists), and the like, thereof, such as, for example, suitable peptides, lipids, proteins, and small molecules. In addition, the present invention provides methods of "de-orphanating" ligands for GPCRs by screening these ligands against the "de-orphanated GPCRs."

All of the documents cited herein, including the foregoing, as well as the documents cited within the mentioned documents, are incorporated by reference herein in their entireties.

### BRIEF DESCRIPTION OF THE DRAWINGS

For a better understanding of the invention as well as other objects and further features thereof, reference is made to the following detailed description of various preferred embodiments thereof taken in conjunction with the accompanying drawings wherein:
FIGURE 1 is a ball-n-stick representation of an aminergic GPCR of the present invention depicting both the aspartic acid in TM3 at position 117 in rhodopsin and the tryptophan in TM7 at position 293 in rhodopsin, in their positions in relation to the ligand binding pocket.

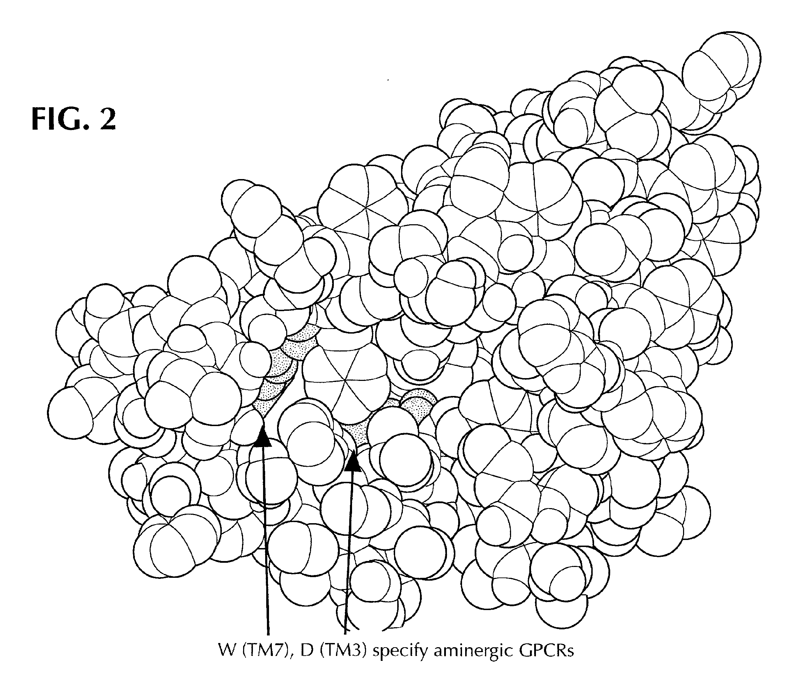

FIGURE 2 is a space-filling model of a folded aminergic GPCR of the present invention depicting both the aspartic acid in TM3 at position 117 in rhodopsin and the tryptophan in TM7 at position 293 in rhodopsin, in their positions in relation to the ligand binding pocket.

### DETAILED DESCRIPTION OF THE INVENTION

Unless otherwise noted, the terms used throughout this specification and the appendant claims generally have their usual meaning as understood by those of ordinary skill in the art. See, for example, Chemical Principles, 4^{th} Edition, by W.L. Masterton and E.J. Slowinski, published in 1977 by W.B. Saunders Company (Philadelphia); Grant & Hackh's Chemical Dictionary, 5^{th} Edition, by Roger Grant and Claire Grant, published in 1987 by McGraw-Hill, Inc. (New York); The Dictionary of Cell & Molecular Biology, 3^{rd} Edition, by Lackie, J.M and Dow, J.A.T., published in 1999 by Academic Press (New York); and Instant Notes in Molecular Biology, by Turner, P.C. *et al*., published in 1998 by BIOS Scientific Publishers Limited. The following terms are intended to have the following general meanings as they are used herein:
"amino acid motif' means a diagnostic tool that comprises particular types of amino acid residues in particular positions in the protein alignment and, as such, permits the identification of members of a subfamily;
"binding" or "binding interaction" refer to the interaction(s) between the GPCR and the ligand(s), e.g., salt bridges, hydrogen bonds, hydrophobic contacts;
"common feature" refers to a structural, chemical, or physical characteristic that enables particular binding interactions unique to a ligand type, such as, for example, a "common chemical moiety" which refers to a chemical characteristic that enables particular binding interactions unique to a ligand type, i.e., serotonin (A), acetylcholine (B), histamine (C), dopamine (D), and epinephrine (E), within the aminergic ligand family, each contain an amino group as depicted below: 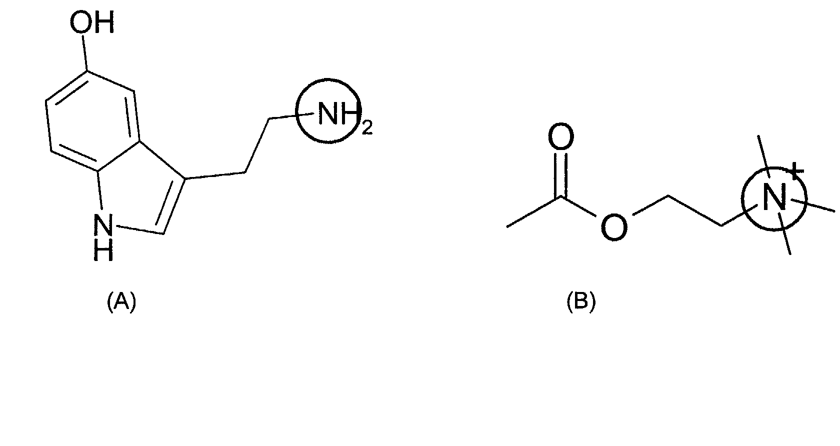 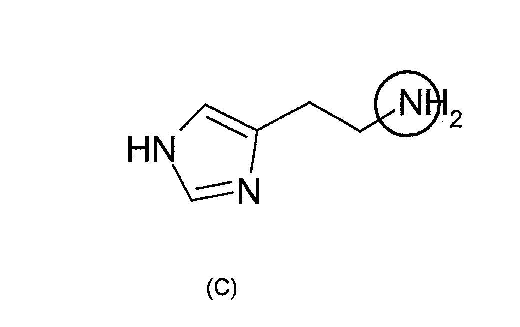 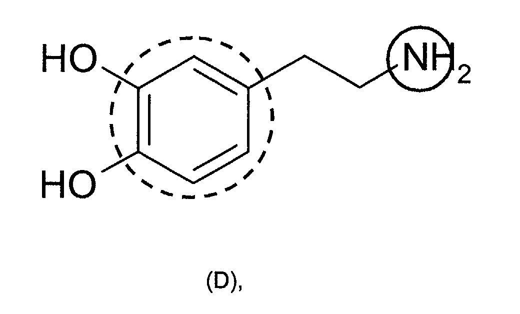 and 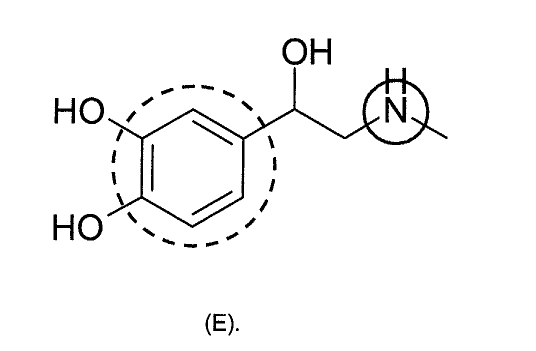
"conserved position" means that substantially the same type of amino acid residue, where type means that the chemical, physical, structural, and sterical characteristics or properties, e.g., shape, charge, aromaticity, or hydrophobicity, is maintained in that position and, in addition, the same type of amino acid residue may be the same amino acid residue;
"conserved residue" means a residue that is maintained in members of, e.g., a subfamily or superfamily;
"electrostatic forces" refers to the interactions or forces between particles caused by their electric charges or electronic configurations (e.g., the spatial arrangement of elements, such as atoms in a molecule, the arrangement of electrons in orbitals (electrons are in orbitals around the atomic nucleus, where the number of electrons and their arrangement account for valency and other properties));
"hydrogen bonds" refer to attractive forces between molecules, arising from the interaction between a hydrogen atom in one molecule and a strongly electronegative atom (N, O, F) in a neighboring molecule, e.g., H atoms and O atoms on different water molecules;
"ion pair" refers to a species made up of a cation and an anion held together by strong electrostatic forces;
"ligand type" refers to the biological, chemical and physical characteristics or properties (e.g., atomic composition and connectivity, electronic configuration (e.g., charge distribution, aromaticity, and similar), hydrophobicity, molecular weight, polarity, products of a common biochemical pathway or process, and shape (e.g., stereochemistry) of an entity that a set of GPCRs (subfamily) binds to, e.g., interactions of ligands and GPCRs involves, for example, hydrogen bonds, ion pairs, and hydrophobic contacts;
"R group" of R-CHNH₂COOH (α-amino acid structure) represents an organic radical, which can range from an H atom to a large aliphatic or aromatic group;
"rhodopsin-like GPCR" means a GPCR having the basic structural elements of rhodopsin, i.e., an extracellular N-terminal segment, seven TMs, which form the TM core, three exoloops, three cytoloops, and a C-terminal segment (for completeness sake, non-rhodopsin-like GPCRs also have these features), and rhodopsin-like GPCRs share certain motifs, like the DRY motif in TM3, and the P in TM7;
"subfamily" means a set of GPCRs that bind a common ligand type, e.g., subfamilies which comprise the rhodopsin-like GPCR superfamily, including, for example, (a) receptors for amines, nucleotides, and lipid molecules; (b) peptide hormone receptors; (c) protease (thrombin) activated receptors; (d) glycoprotein hormone receptors (LH, FSH, hCG, TSH); and (e) neurotransmitter receptors (Ca⁺⁺, glutamate, GABA); and
"superfamily" means a family including all rhodopsin-like GPCRs, e.g., the Class A Superfamily comprises aminergic (bioaminergic), cannabinoid, glycoprotein hormone, lysophingolipid, melatonin, nucleotide, olfactory, opsin, peptide, and "orphan" subfamilies; the aminergic subfamily comprises acetylcholine (muscarinic) receptors, adrenergic (alpha, beta) receptors, dopamine receptors, histamine receptors, and serotonin (5-hydroxytryptamine) receptors; the lipid subfamily comprises eicosanoids (leukotrienes (e.g., LTB, LTC) and prostanoids), lysophingolipids and lysophosphatidylcholine; the nucleotide subfamily comprises adenosine, nucleoside-sugar, P2U, and P2Y; the peptide subfamily comprises angiotensin, apalin, bombesin, bradykinin, chemokine (e.g., CC, CXC, FMLP, interleukin, anaphylatoxin), cholecystokinin, endothelin, galanin, melanocortin, motilin, neuropeptide (e.g., NPFF, neuropeptide Y), neurotensin, opioid, orexin, other peptides (e.g., KISS), proteinase-activated, somatostatin, tachykinin, urotensin, and vasopressin.

Unless otherwise noted, throughout this description and the appendant claims: asn is asparagine (slightly hydrophilic R group, little influence on water solubility); asp is aspartic acid (hydrophilic R group, enhance water solubility); arg is arginine (hydrophilic R group, enhance water solubility); BLAST refers to Basic Local Alignment Search Tool, a sequence similarity alignment algorithm; cys is cysteine (slightly hydrophilic R group, little influence on water solubility); FSH is follicle-stimulating hormone; hCG is human chorionic gonadotrophin; LBD is ligand binding domain; LH is luteinizing hormone; phe is phenylalanine (hydrophobic R group, decrease solubility in water); pro is proline (slightly hydrophilic R group, little influence on water solubility); ser is serine (slightly hydrophilic R group, little influence on water solubility); TM is transmembrane; trp is tryptophan; TSH is thyroid-stimulating hormone; and tyr is tyrosine (slightly hydrophilic R group, little influence on water solubility).

As those skilled in the art will appreciate, GPCRs generate and mediate the transduction of several different signals from the cell surface to sites within the cell. Mutations in GPCRs have been shown to be related to certain hereditary and somatic disorders and diseases. Some of these mutations have been reported to be beneficial (e.g., mutations in CCR5), while some of these mutations have been reported to be non-beneficial (e.g., preclude ligand binding, constitutively generate signals, are not suitably expressed on the cell surface, and similar).

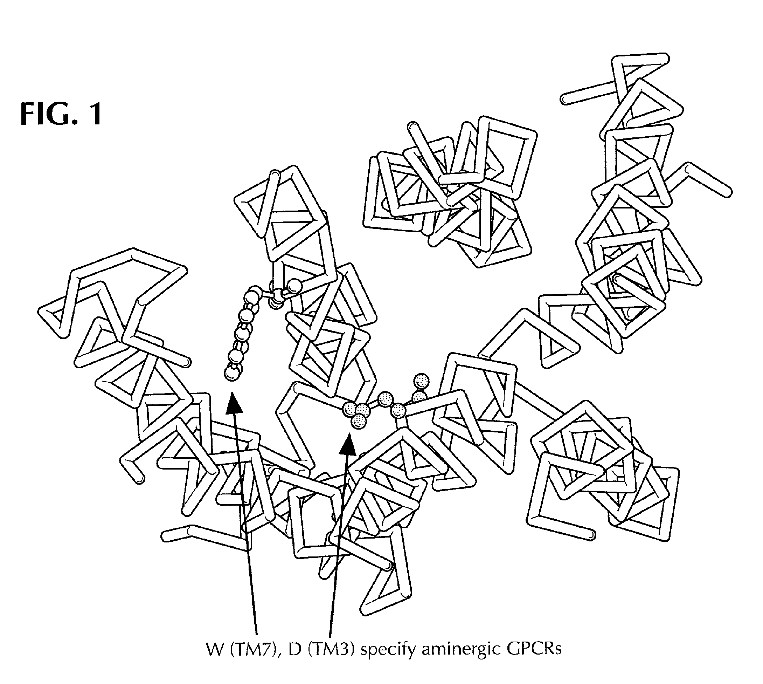

Using, for example, sequence homology, ligand structure and receptor function, GPCRs have been classified into more than 100 subfamilies, the members of which show substantial amino acid homology. See, for example, the article by T.H. Ji *et al*., "G Protein-coupled Receptors: I. Diversity of Receptor-Ligand Interactions," Minireview, *J. Biol. Chem*. 273 (28): 17299-17302 (1998) and, in particular, Fig. 1 on page 17300, as well as the references cited therein.

The general structure of a Class A (rhodopsin-like) GPCR has an extracellular N-terminal segment, seven TMs, which form the TM core, three exoloops, three cytoloops, and a C-terminal segment. See, e.g., aforementioned Fig 1 of T.H. Ji *et al*. (1998). A fourth cytoplasmic loop is formed when the C-terminal segment is palmitoylated at cysteine (cys). See, e.g., T.H. Ji *et al*. (1998). GPCRs have been classified by the type of ligand(s) that they interact with, e.g., GPCRs for amines, nucleotides, and lipid moieties; GPCRs for peptide hormones; GPCRs that are activated by proteases; GPCRs for glycoprotein hormones; and GPCRs for neurotransmitters.

Several Class A (rhodopsin-like GPCRs) have been reported, and include, for example, aminergic: acetylcholine (muscarinic acetylcholine receptors M1, M2, M3, M4, and M5), adrenergic (Alpha-1A, Alpha-1B, Alpha-1D, Alpha-2A, Alpha-2B, Alpha-2C-1, Beta-1, Beta-2, Beta-3), dopamine (D(1A), D(1B), D(2), D(3), D(4)), histamine (H1, H2, H3, H4), serotonin ( 5-HT-1A, 5-HT-1B, 5-HT-1D, 5-HT-1E, 5-HT-1F, 5-HT-2A, 5-HT-2B, 5-HT-2C, 5-HT-4, 5-HT-5A, 5-HT-6, 5-HT-7), cannabinoid (CB1, CB2), glycoprotein hormone (follicle stimulating hormone receptor (FSH-R), GPR24 melanin concentrating hormone receptor, lutropin-choriogonadotropic hormone receptor (LSH-R), GPCR0459 Melanin-concentrating hormone receptor 2 (MCH2), thyrotropin receptor (TSH-R)), lipid (eicosanoid (leukotriene (LTB (leukotriene B4 receptor (aka P2Y purinoceptor 7, P2Y7), leukotriene B4 receptor (aka Fishboy G-protein coupled receptor), and LTC (cysteinyl leukotriene receptor CysLT2), cysteinyl leukotriene receptor (CYSLT1)), prostanoid (CRTH2 (GPR44), prostacyclin receptor (prostanoid IP receptor), prostaglandin D2 receptor (prostanoid DP receptor), prostaglandin E2 receptor EP1 subtype (prostanoid EP1 receptor), prostaglandin E2 receptor EP2 subtype (prostanoid EP2 receptor), prostaglandin E2 receptor EP3 subtype (prostanoid EP3 receptor), prostaglandin E2 receptor EP4 subtype (prostanoid EP4 receptor), prostaglandin F2-alpha receptor (prostanoid FP receptor), thromboxane A2 receptor (TXA2-R) (prostanoid TP receptor)), lysophingolipid (EDG-4, EDG-1, EDG6, EDG-7, EDG-2, EDG-3, EDG5, EDG-8), sphingosylphosphorylcholine (OGR1), lysophosphatidylcholine (G2A)), melatonin (H9, MEL-1A-R, MEL-1B-R)), nucleotide (P2Y12 platelet ADP receptor), adenosine (A1, A2A, A2B, A3), nucleoside-sugar KIAA0001, UDP-Glucose), P2U (P2U1, P2Y2, P2Y1, P2Y11, P2Y6), olfactory (OR1A2, OR1A1, Olfactory receptor 17-90, OR17-24, 6M1-16*01/02/03, 6M1-18*01/02, 6M1-4P*02/05, Olfactory receptor 89, AC006271, AF143328, AL096770-01, AL096770-02, AL096770-03, AL096770-04, AL121944, AL135841, BC62940_2, BC85395_1, BC85395_3, F20569_1, F20722_1, F20722_2, FAT11, GPR1, GRIR-1, OR17-4, HGMP07I, HGMP07J, HI7, HOR 5' beta, HOR 5' beta, HOR3'beta1, HPFH1OR, HS6M1-1, HS6M1-3, HS6M1-6, HSA1, HSA10, HSA3, HSA5, HSA8, OR16-35, H_DJ0855D21.1, H_DJ0988G15.2, JCG2, OLF1, OLF3, OLF4, OLFR 42B, OLFR42B, OLRCC15, OR1-25, OR1-26, OR10A1, OR17-201, OR17-209, OR17-210, OR17-219, OR17-228, OR17-30, OR17-40, OR2C1, OR2D2, OR5-40, OR5D3, OR5F1, OR6A1, OR7-138, R30385_1, TPCR100, TPCR110, TPCR120, TPCR16, TPCR24, TPCR25, TPCR26, TPCR27, TPCR85, TPCR92, Z98744, dJ25J6.1, dJ88J8.1, prostate specific olfactory receptor, putative taste receptor HTR2, opsin (blue-sensitive opsin, encephalopsin, green-sensitive opsin, melanopsin, RPE-retinal G protein-coupled receptor, red-sensitive opsin, rhodopsin, visual pigment-like receptor, peropsin), peptide (angiotensin (AT-1, AT2), apalin (APJ. Apelin receptor), bombesin (BRS-3, GRP-R (GRP-preferring bombesin receptor), neuromedin-B receptor, NMB-R (neuromedin-B-preferring bombesin receptor)), bradykinin (BK-1 receptor, BK-2 receptor), chemokine (CC (CCR1, CCR10, CCR11, CCR2, CCR4, CCR5, CCR6, CCR7, CCR8, CCR9, CX3CR1, XCR1, CXCR3, CXCR4, CXCR5, FMET-LEU-PHE receptor (FMLP receptor), FMLP-related receptor I (FMLP-R-I), FMLP-related receptor II (FMLP-R-II), interleukin (CXCR1, CXCR2), anaphylatoxin (C3A -R, C5A-R)), cholecystokinin (CCK-A receptor, CCK-B receptor), endothelin (ET-B, ET-A), galanin (GAL1-R, GAL2-R, GAL3-R), melanocortin (MC1-R (MSH-R), MC2-R (ACTH-R), MC3-R, MC5-R)), motilin (GPR38 (Motilin Receptor)), neuropeptide (NPFF (NPFF2, RFamide-related peptide receptor), neuropeptide Y (NPY1-R, NPY2-R, NPY4-R, NPY5-R, NPY6-R), neurotensin (NTR1, NTR2), opioid (DOR-1, KOR-1, MOR-1, nociceptin receptor, KOR-3), orexin (orexin receptor type 1, OX1R (hypocretin receptor type 1), OX2R (Hypocretin receptor type 2)), other peptide (KiSS receptor (GPR54)), proteinase activated (PAR-2, PAR-3, PAR-4, thrombin receptor), somatostatin (SS5R, SS1R, SS2R, SS3R, SS4R), tachykinin (NK-3 receptor, NK-4 receptor, NMU1R (aka FM3), NMU2R, NK-2 receptor, NK-1 receptor), urotensin (Urotensin II receptor, GPR14), vasopressin (OT-R, vasopressin V1A receptor, vasopressin V1B receptor, vasopressin V2 receptor), platelet activating factor (leukocyte platelet-activating factor receptor, platelet activating factor receptor (PAF-R)), releasing Hormone (GNRH-R, GHS-R, Prolactin-releasing peptide receptor (GPR10), thyrotropin-releasing hormone receptor (TRH-R), Type II GnRH-R protein)).

Class B (e.g., "orphan," secretin (CRH, V1P)), C (e.g., metabotropic, GABA-B), F (e.g., frizzled, frizzle-like, frizzle homologs), and taste GPCRs have also been identified.

Class B "orphan GPCRs" include, for example, cadherin EGF LAG seven-pass G-type receptor (CELSR1), cell surface glycoprotein EMR1, class B G protein-coupled receptor Y91625, EGF-like module containing mucin-like receptor EMR3, flamingo 1 (FMI1), EMR2, FLJ14454, KIAA1828, AL033377 (HE6 homolog), ETL, GPR56, HE6, KIAA0758, latrophilin-1, latrophilin-2, latrophilin-3, VLGR1). Those skilled in the art will appreciate, based on the present description, how to use the methods of the invention to "de-orphanate" these "orphan GPCRs" as well.

Class B GPCRs further include, e.g., secretins (BAI-1, BAI-2, BAI-3, calcitonin gene-related peptide type 1 receptor, calcitonin receptor (CT-R), GIP-R, glucagon receptor (GL-R), glucagon-like peptide 1 receptor (GLP-1 receptor), glucagon-like peptide-2 receptor (GLP2R), growth hormone-releasing hormone receptor (GHRH receptor), leucocyte antigen CD97, ocular albinism type 1 protein, PTH2 receptor, PTHR receptor, PACAP-R-1, FMI1 (MEGF2), SCT-R, CRH (CRF1, CRF2), VIP (VIP-R-1, VIP-R-2).

Class C GPCRs include, for example, metabotropic (CASR, metabotropic glutamate receptor 1, metabotropic glutamate receptor 2, metabotropic glutamate receptor 3, metabotropic glutamate receptor 4, metabotropic glutamate receptor 5, metabotropic glutamate receptor 6, metabotropic glutamate receptor 7, metabotropic glutamate receptor 8, sensory transduction G-protein coupled receptor-B3, taste receptor GPCR-B4, and GABA-B (GABA-B1A receptor, GABA-B2 receptor).

Class F GPCRs include, e.g., frizzled 1 transmembrane receptor, frizzled 10 transmembrane receptor, frizzled 2 transmembrane receptor, frizzled 3 transmembrane receptor, frizzled 4 transmembrane receptor, frizzled 5 transmembrane receptor, frizzled 6 transmembrane receptor, frizzled 7 transmembrane receptor, frizzled 9 transmembrane receptor, frizzled-like receptor smoothened homolog (SMO), and frizzled-7 homologue.

Taste GPCRs include, e.g., T2R1, T2R10, T2R13, T2R14, T2R16, T2R3, T2R4, T2R5, T2R7, T2R8, and T2R9.

For several GPCRs, classification by ligand structure has not yet occurred because no ligands have been identified that bind to these "orphan GPCRs." Classification of GPCR sequences and prediction of their natural ligands is important for identifying and validating new GPCR targets. GPCRs present imposing challenges for bioinformatics approaches due to poor sequence conservation, particularly outside of the TM regions, as well as the lack of three-dimensional structural information other than that which exists for bovine rhodopsin. "Orphan GPCRs" can be classified to identify the most likely subfamily for each "orphan GPCR" sequence based on sequence motifs, subfamily statistical profiles, three-dimensional modeling, and hierarchical clustering. Models can be further validated by site-directed mutagenesis, and such models will enhance the ability of those skilled in the art to predict structure-activity and structure-specificity relationships.

Class A "orphan GPCRs" that have been reported include, for example, 5-hydroxytriptamine receptor homologue, transmembrane receptor HEOAD54, chemokine receptor, chemokine receptor-like 1, G-protein-coupled receptor DEZ, chemokine receptor-like 2, IL-8-related receptor DRY12 GPR30 CEPR, dorsal root receptor 1 DRR1, dorsal root receptor 2 DRR2, dorsal root receptor 3 DRR3, dorsal root receptor 4 DRR4, dorsal root receptor 5 DRR5, dorsal root receptor 6 DRR6, Duffy antigen, EBV-induced G protein-coupled receptor 2 (EBI2), EDG homologue, EDG homologue (GPR45), EDG-homologue, GPR35, GPR37, GPR75, G protein-coupled receptor (RAIG1), BONZO (STRL33), D38449, ETBR-LP-2, GPR1, GPR12, GPR15, GPR17, GPR18, GPR19, GPR20, GPR22, GPR3 (ACCA "orphan" receptor), GPR31, GPR32, GPR34, GPR39, GPR4 (GPR19), GPR40, GPR41, GPR43, GPR55, GPR6, GPR7, GPR73, GPR8, HG38, HM74, LGR4, RDC1 homolog, GPR48, GPR61, GPR62, GPR77, GPR84, GPR86, GPR87, GPR72, GPRC5B, H7TBA62, G-protein coupled receptor R97222, SALPR, Y13583, Y36302, GPR58, GPR57, RE2, GPR21, GPR52, SREB1, SREB2, SREB3, LGR7, MAS protooncogene, MAS-related G protein-coupled receptor MRG, neurotensin receptor ntr2 receptor homologue, GPR25, H963, P2Y10, P2Y5, P2Y9, FMLP related receptor homolog, pheromone receptor homologue, N-formyl peptide receptor homolog, GPR92, RAIG1 homolog, FKSG46, FKSG47, V1RL1, CRAM-A, FKSG80, seven transmembrane-domain protein p40 homologue TASP testis specific adriamycin sensitivity protein, striatum-specific G protein-coupled receptor, T cell-death associated protein, and thoracic aorta G-protein coupled receptor. Those skilled in the art will understand how to use, for example, common BLAST programs, e.g., WASHU, to gain more information about each of the "orphan GPCRs" referred to herein. Additionally, those skilled in the art will understand, based on the present description, how to use the methods of the invention to "de-orphanate" each, any, and all of such "orphan GPCRs," and any other non-listed "orphan GPCRs." Identification of a subfamily-specific motif according to a preferred embodiment of the present invention comprises the steps of: performing a multiple sequence alignment of known Class A (rhodopsin-like) GPCRs, scanning down the remaining alignment positions, marking residues (or residue classes) conserved in a particular subfamily of GPCRs, setting aside a residue (or class of residues) that is not also characteristic of the Class A Superfamily for consideration as a ligand-binding residue, evaluating conserved polar, charged, and aromatic amino acid residues, especially those within the transmembrane domains, as determinants of ligand-binding specificity for the subfamily, and disregarding regions of the alignment that fall within the intracellular portion of the receptor, including the three intracellular domains and the C-terminal domain in their entirety, as well as, portions of each of the TM domains.

The binding of ligands to their receptors is often specific because of electronic interactions, manifested as hydrogen-bond pairs, ionic bonds, and aromatic interactions. Even if aliphatic amino acids actually touch the ligand within a transmembrane pocket, they often are not suitable as part of discrimination motif because the hydrophobic residues are commonly seen within the helical regions.

Regions of the alignment that fall within the intracellular portion of the receptor are not included in the subfamily-specific as they are unlikely to indirect directly with the cognate ligand, which is typically presented to the receptor from the extracellular face of the receptor.

The putative role of these amino acid residues can be supported by site-directed mutagenesis experiments. As those skilled in the art will appreciate, where one observes adverse effects on ligand binding and/or activation after replacement of an implicated residue, it is more likely that it plays a direct role in ligand binding. This step is important to distinguish residues conserved in a subfamily due to common ancestry from those that are conserved due to functional constraints.

The physico-chemical properties of the conserved amino acid (or type) are then optionally correlated with shared physico-chemical properties of the ligand type. For example, where an amino acid conserved in the subfamily is positively charged, it would be useful to propose a negatively charged moiety in the ligand that interacts with the conserved residue. Successful correlation of these data also lends support to the hypothesis that a given residue, or set of residues, is responsible for ligand specificity, but is not necessary for a position to be considered in the final discrimination motif.

Finally, all implicated positions and their residue identities (or class) are collected, forming a final set from which to build a discrimination motif for the subfamily for refinement and evaluation for sensitivity and selectivity. One approach is simply to search exhaustively over all combinations of residue (or residue types) to optimize selectivity and sensitivity. Alternatively, one can select the position that is conserved throughout the subfamily and has minimal representation in other subfamilies. Where this residue is absent in all other subfamilies, this amino acid may in itself constitute a subfamily motif. However, where this residue, or residue class, is seen in the same position in other subfamilies, one adds other positions that are also completely conserved in the subfamily but are increasingly common in other subfamilies. After each subsequent addition, the emerging motif is assessed for specificity. This iterative refinement procedure would terminate when a motif is constructed that describes the subfamily of interest without also matching any other sequence of another subfamily. A stepwise addition of additional conserved positions is desirable to optimize sensitivity of the motif without sacrificing specificity. Avoiding positions not supported by mutagenesis data also minimizes the risk of adding to the motif residues unrelated to ligand binding.

The methods provided by the present invention comprise the steps of: aligning members of a subfamily of interest, making position-by-position observations, building and validating three dimensional models, and converting the models to a sequence motifs, e.g., for classifying "orphan" receptors. The position-by-position observations include, for example, identifying which residues are conserved, whether the same residues are also conserved in the Class A Superfamily, and whether the physicochemical distinctions are substantially justifiable by the ligand type.

The present invention is illustrated by the following EXAMPLES. The foregoing and following description of the present invention and the various embodiments are not intended to be limiting of the invention but rather are illustrative thereof. Hence, it will be understood that the invention is not limited to the specific details of these EXAMPLES. For instance, those skilled in the art will understand and appreciate from these EXAMPLES, based on the present description, how to apply the methods of the invention to determine an amino acid sequence motif for each and any of the rhodopsin-like GPCR subfamilies, and to use such a motif to "de-orphanate" an "orphan GPCR" belonging to the selected subfamily.

### EXAMPLE I

### Identification of Aminergic GPCR Amino Acid Sequence Motif

First, thirty-three (33) of the thirty-four (34) known GPCRs of the aminergic subfamily were selected. By hand-aligning these sequences, the twenty (20) residues conserved in all of these aminergic GPCRs were identified (with the structural location indicated) and numbered according to the corresponding residue number in the reference GPCR rhodopsin (numbered from N-terminus to C-terminus) as provided in Table 5 below.

**Table 5**

| **TM1** | **TM2** | **EC1** | **TM3** | **TM4** | **EC2** | **TM5** | **TM6** | **TM7** |
|---|---|---|---|---|---|---|---|---|
| Asn | Asp | Trp | Cys | Trp | Cys | Phe | Phe | Trp |
| 55 | 83 | 103 | 110 | 161 | 187 | 212 | 261 | 293 |
| | | | Asp | | | Pro | Trp | Ser |
| | | | 117 | | | 215 | 265 | 299 |
| | | | Ser | | | | Pro | Asn |
| | | | 124 | | | | 267 | 302 |
| | | | Asp | | | | | Pro |
| | | | 134 | | | | | 303 |
| | | | Arg | | | | | Tyr |
| | | | 135 | | | | | 306 |

Second, the identified conserved residues depicted in Table 5 were compared with residues conserved across the entire GPCR Class A Superfamily, and the commonly conserved residues removed from the putative aminergic subfamily sequence motif, as illustrated in Table 6 below which provides the remaining residues.

**Table 6**

| **TM1** | **TM2** | **EC1** | **TM3** | **TM4** | **EC2** | **TM5** | **TM6** | **TM7** |
|---|---|---|---|---|---|---|---|---|
| | | Trp | | | | Phe | Phe | Trp |
| | | 103 | | | | 212 | 261 | 293 |
| | | | Asp | | | | Trp | Ser |
| | | | 117 | | | | 265 | 299 |
| | | | Ser | | | | | Asn |
| | | | 124 | | | | | 302 |
| | | | Asp | | | | | |
| | | | 134 | | | | | |
| | | | | | | | | Tyr |
| | | | | | | | | 306 |

Third, the identified conserved residues depicted in Table 6 that are structurally located in the intracellular portions of the GPCR and, as such, are less likely to interact with the ligand, were removed from the putative aminergic subfamily sequence motif, as illustrated in Table 7 below which provides the remaining residues.

**Table 7**

| **TM1** | **TM2** | **EC1** | **TM3** | **TM4** | **EC2** | **TM5** | **TM6** | **TM7** |
|---|---|---|---|---|---|---|---|---|
| | | Trp | | | | Phe | Phe | Trp |
| | | 103 | | | | 212 | 261 | 293 |
| | | | Asp | | | | Trp | Ser |
| | | | 117 | | | | 265 | 299 |
| | | | Ser | | | | | |
| | | | 124 | | | | | |

Fourth, the remaining residues depicted in Table 7 were evaluated for their relative representation in non-aminergic subfamilies of the GPCR Class A Superfamily (in parentheses after the residue) and ranked least representative (more aminergic specific) to most representative (less aminergic specific), as provided in Table 8 below.

**Table 8**

| **TM1** | **TM2** | **EC1** | **TM3** | **TM4** | **EC2** | **TM5** | **TM6** | **TM7** |
|---|---|---|---|---|---|---|---|---|
| | | **#5** | | | | **#3** | **#8** | **#2** |
| | | (97) | | | | (81) | (115) | (14) |
| | | Trp | | | | Phe | Phe | Trp |
| | | 103 | | | | 212 | 261 | 293 |
| | | | **#1** | | | | **#7** | **#4** |
| | | | (11) | | | | (106) | (104) |
| | | | Asp | | | | Trp | Ser |
| | | | 117 | | | | 265 | 299 |
| | | | **#6** | | | | | |
| | | | (104) | | | | | |
| | | | Ser | | | | | |
| | | | 124 | | | | | |

As those skilled in the art will appreciate from the data shown in Table 8, the conserved residue of the examined aminergic GPCRs that is least represented in non-aminergic GPCRs is the aspartic acid in TM3 at position 117 in rhodopsin, with the tryptophan in TM7 at position 293 in rhodopsin next in line.

The negatively-charged side chain of the aspartic acid 117 residue can interact with the positively-charged amine groups of the ligand bioamines. In fact, as mentioned earlier, site-directed mutagenesis of this aspartic acid residue has been reported to affect ligand binding. Likewise, site-directed mutagenesis has also confirmed that the tryptophan 293 residue can interact with the amine group of an aminergic ligand via an amine-aromatic interaction.

Terminating the stepwise addition of residues to the motif after sufficient residues have been added to distinguish the subfamily from all other GPCRs guarantees maximum motif sensitivity.

Despite the reported mutagenesis data, those skilled in the art will understand and appreciate that a complete motif that optimally satisfies the sensitivity and specificity criteria for identifying members of the aminergic GPCR subfamily has yet to be identified. Emphasis on residues important to ligand binding, based on mutation data, correlation of ligand and residue properties, and location on the extracellular face of the GPCR, assures that conserved amino acids assigned to the motif were involved in binding the ligand type of the GPCR subfamily. Defining the motif in this way increases the likelihood that the motif will remain specific for the GPCR subfamily as more GPCRs that bind its ligand type are identified in various ways.

As shown in this Example, the present invention provides a sensitive and specific aminergic sequence motif, i.e., the combination of the conserved aspartic acid in TM3 and the conserved tryptophan in TM7, a combination which is not present in any known non-aminergic GPCRs.

### EXAMPLE 2

### Use of the Aminergic GPCR Amino Acid Sequence Motif to "De-orphanate" an "Orphan GPCR"

Eight known "orphan GPCRs" were selected for possible aminergic assignment using the aminergic motif of the invention provided in Example 1 hereinabove, namely, GPCR0441 (see, e.g., WO00/60081) and GPCR0503 (see, e.g., WO00/60081).

The superfamily motifs of these sequences, i.e., the DRY in TM3 (downstream of the D) and the NP..Y motif in TM7 (near the W), were first aligned.

These "orphan GPCRs" were then examined for the presence of an aspartic acid in TM3 at position 117 in rhodopsin, which proved to be present in each case.

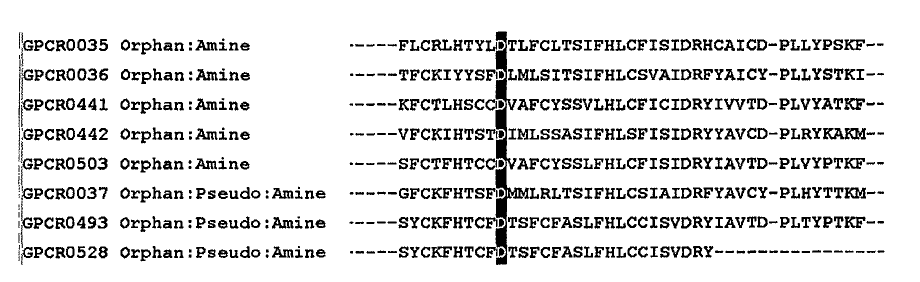

Within each of the above sequences, the D was located in TM3 at position 117 which, for reference, given that these GPCRs are of different overall length, corresponds to the following positions within the actual GPCR amino acid sequences: 114 (0035), 78 (0036), 111 (0441), 103 (0442), and 112 (0503).

The "orphans" were then further examined for the presence of a tryptophan in TM7 at position 293 in rhodopsin, which proved to be present in each case.

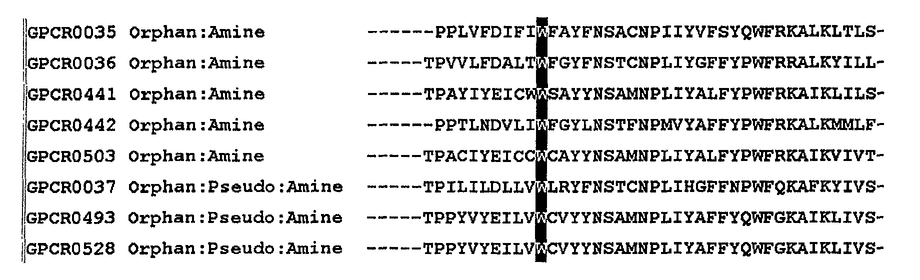

Within each of the above sequences, the W was located in TM7 at position 117 which, for reference, given that these GPCRs are of different overall length, corresponds to the following positions within the actual GPCR amino acid sequences: 292 (0035), 257 (0036), 297 (0441), 291 (0442), and 299 (0503).

Hence, using the motif of the present invention, since only aminergic GPCRs have both of these key distinguishing residues, these eight "orphans" may be assigned to the aminergic subfamily.

Further confirmation of these subfamily assignments can be made, where so desired, by using any suitable methods therefor, e.g., conventional functional assays such as, for example, ligand binding assays using know aminergic ligands. Those skilled in the art will understand, based on the present description, how to devise and to perform such assays.

For example, both GPCR0441 and GPCR0503 have been now reported to be aminergic GPCRs, e.g., see WO00/60081 which describes that these GPCRs are trace amine receptors, a type of aminergic GPCR.

As stated earlier hereinabove, the aminergic subfamily of GPCRs includes receptors for histamine. Ohta *et al*., in their article "Molecular Cloning and Characterization of a Novel Type of Histamine Receptor Preferentially Expressed in Leukocytes," published in the *J. Biol. Chem*. 275 (47): 36781-36786 (2000), disclosed the molecular cloning of a novel histamine receptor (AB044934), a type of aminergic GPCR, classified as a histamine receptor, in part, on the basis of amino acid sequence homology with known histamine receptors, and functional activation of the subject receptor by transiently expressing the target in 293-EBNA (Epstein-Barr virus nuclear antigen) cells.

By contrast, had Ohta *et al*. been able to use the novel motifs provided by the present invention, after aligning the DRY and NP..Y superfamily features, Ohta could have assigned the novel GPCR to the aminergic subfamily, avoiding the need to perform the functional assays.

As illustrated by the data provided hereinabove, the motifs and methods of the invention show that such motifs can be identified and then used, for example, to "de-orphanate" "orphan GPCRs" by assigning them to their rightful subfamily of the Class A superfamily.

Although these examples are directed to identifying and confirming a sequence motif for the aminergic GPCR subfamily, one skilled in the art will recognize that the same methods may be applied to determine and confirm sequence motifs for other GPCR subfamilies.

## Claims

1. A method for determining amino acid sequence motifs characteristic of GPCR subfamilies, where the subfamily members interact with members of a ligand family through the identified motifs, comprising: (a) manually aligning amino acid sequences of said members of a GPCR subfamily to create a subfamily alignment, (b) comparing said subfamily alignment with a known GPCR superfamily alignment, (c) identifying at least one conserved position in said subfamily that is not conserved in said superfamily assignment, (d) identifying at least one common feature (e.g., a common chemical moiety) in members of said ligand family, and (e) determining if a binding interaction exists between said conserved position in said subfamily of (c) and said common feature of (d), where the presence of said binding interaction indicates that said conserved position of said subfamily is part of said sequence motif characteristic of said subfamily.

2. A method of determining amino acid sequence motifs characteristic of GPCR subfamilies, comprising: (a) manually aligning amino acid sequences of members of the selected GPCR subfamily to create a subfamily alignment, (b) comparing the subfamily alignment with a known GPCR superfamily alignment, and (c) identifying at least one conserved position in the subfamily that is not conserved in the superfamily assignment, thus providing at least one distinguishing characteristic of the subfamily with respect to the superfamily.

3. A method of determining whether an orphan GPCR belongs to a GPCR subfamily, comprising: (a) manually aligning amino acid sequences of members of the selected GPCR subfamily to create a subfamily alignment, (b) comparing the subfamily alignment with a known GPCR superfamily alignment, (c) identifying at least one conserved position in the subfamily that is not conserved in the superfamily assignment, and (d) determining whether the orphan GPCR comprises the subfamily's conserved position, thus identifying the orphan GPCR as a member of the subfamily.

4. A method of determining whether an orphan GPCR belongs to a GPCR subfamily, where said subfamily members interact with members of a ligand family through identified motifs, comprising: (a) manually aligning amino acid sequences of members of a GPCR subfamily to create a subfamily alignment, (b) comparing said subfamily alignment with a known GPCR superfamily alignment, (c) identifying at least one conserved position in said subfamily that is not conserved in said superfamily assignment, (d) identifying at least one common feature said members of said ligand family, (e) determining if a binding interaction exists between said conserved position in the subfamily of (c) and said common feature of (d), where the presence of a binding interaction indicates that said conserved position of said subfamily is part of said sequence motif characteristic of the subfamily, and (f) determining whether said orphan GPCR comprises said sequence motif characteristic of said subfamily.

5. The method as defined in one of claims 1 to 4, wherein said conserved positions is located on an extracellular portion of the GPCRs of the subfamily.

6. The method as defined in claim 5, wherein said extracellular portion is selected from the group consisting of: the N-terminal domain, an extracellular loop, an extracellular portion of a helix, and a transmembrane helix.

7. The method as defined in one of claims 1 to 4, wherein said conserved position is occupied by a polar or an aromatic amino acid, which is charged or uncharged.

8. The method as defined in claim 7, wherein said aromatic amino acid is selected from the group consisting of: phenylalanine, tyrosine, tryptophan, and histidine.

9. The method as defined in claim 8, wherein said aromatic residue is tryptophan.

10. The method as defined in one of claims 1 or 4, wherein said members of said ligand family are identified by a common property.

11. The method as defined in claim 10, wherein said common property is selected from the group consisting of: atomic composition and connectivity, electronic configuration, hydrophobicity, molecular weight, polarity, products of a common biochemical pathway or process, and shape.

12. The method as defined in one of claims 1 or 4, wherein said ligand family is selected from ligand families that interact with GPCRs.

13. The method as defined in one of claims 1 or 4, wherein said ligand family is selected from the group consisting of: amines, peptides, lipids, melatonins, nucleotides, olfactory ligands, and opsins.

14. The method as defined in one of claims 3 or 4, wherein said binding interaction is determined by interacting a GPCR with a member of a ligand family under conditions favoring ligand binding to said GPCR, exposing the GPCR/ligand complex to conditions favoring crystallization of said complex, and identifying a point of interaction between said GPCR and said member of said ligand family by examining the crystallized complex.

15. The method as defined in one of claims 3 or 4, wherein said binding interaction is determined by site-directed mutagenesis of said GPCR or said member of said ligand family.
